(19) Europäisches Patentamt
European Patent Office
Office européen des brevets

(11) EP 1 219 239 A1

(12) EUROPEAN PATENT APPLICATION

(43) Date of publication:
03.07.2002 Bulletin 2002/27

(51) Int Cl.7: A61B 5/103

(21) Application number: 00128769.7

(22) Date of filing: 30.12.2000

(84) Designated Contracting States:
AT BE CH CY DE DK ES FI FR GB GR IE IT LI LU MC NL PT SE TR
Designated Extension States:
AL LT LV MK RO SI

(71) Applicant: Istituti Ortopedici Rizzoli
40136 Bologna (IT)

(72) Inventor: Martelli, Sandra
40139 Bologna (IT)

(74) Representative: Celestino, Marco
ABM, Agenzia Brevetti & Marchi,
Viale Giovanni Pisano, 31
56123 Pisa (IT)

# (54) Method and apparatus for simultaneous anatomical and functional mapping of a joint

(57) A new method for acquisition and computer elaboration of joint anatomy and motion is provided. The method uses a commercial electrogoniometer and a software for numerical interpolations and interactive display of the anatomical structures during the joint motion. The acquisition protocol and the computer elaboration are easy to execute. Geometrical and functional analysis of the knee are possible. In particular it is possible to reproduce in a controllable and measurable environment the real joint, and to quantify both anatomical and functional features, as well as the correlation to each other. Moreover, tracking anatomical structures is achieved during motion, with numerical and statistical evaluation of adjacent sections and orientations comprising bone, ligaments insertions and interpolated points such as FF centres.

Fig. 1

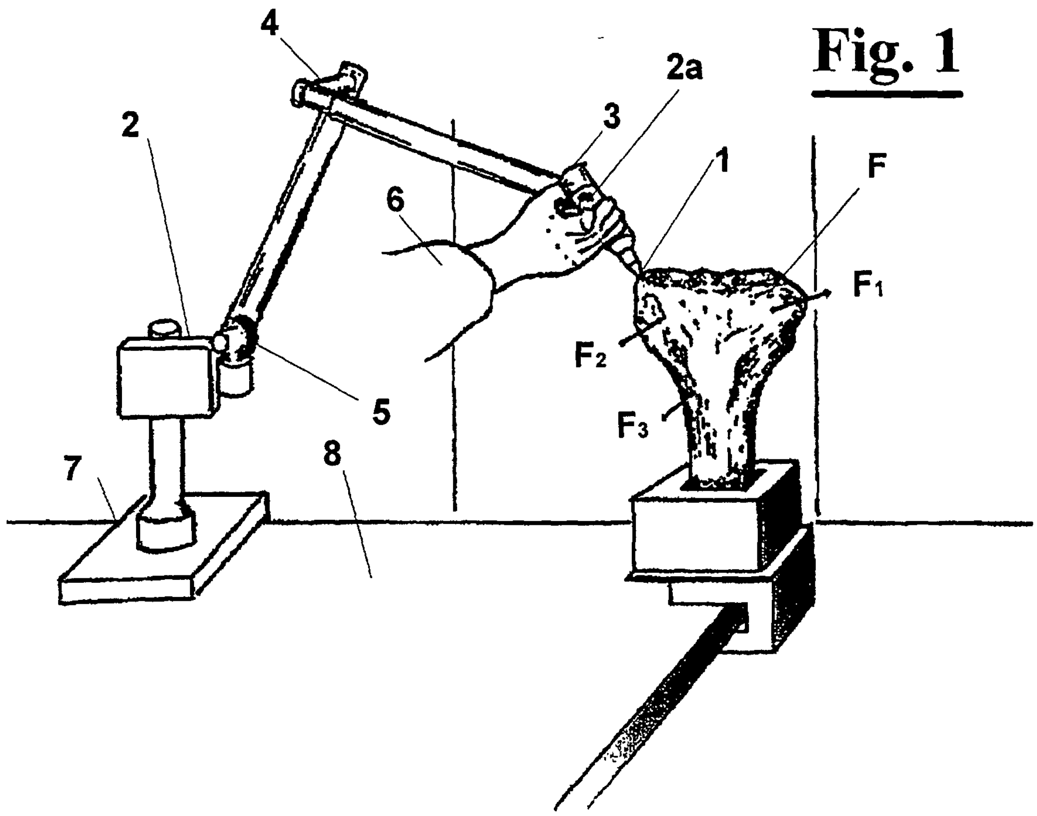


EP 1 219 239 A1

## Description

Field of the invention

The present invention relates to a method for simultaneous anatomical and functional mapping of a joint, such as a knee, elbow, shoulder, hip joint, etc. The method carries out both acquisition and computer elaboration of the joint anatomy and motion.

The kind of joint exemplified hereinafter is a knee joint. However, it is clear that the invention is applicable to other joints, such as elbow, shoulder, hip joints.

Background of the invention

Many anatomical and kinematical studies of joints of the human body have been made. The efficiency in determining the anatomical sizes and shapes, but also in motion tracking, has greatly benefited from technological advancements of the acquisition equipment.

Concerning the knee joint many studies have been made [1, 2, 3]. Nowadays submillimetric mechanical and intrinsic accuracy is possible by using both modern radiographic images [4, 5, 6] and spatial linkages [7, 8, 9, 10, 11, 20, 25, 26].

Techniques are also known, designed for both anatomical and motion acquisition or analysis, that have solved the problem of verifying the relationship between anatomical and kinematic features of the knee joint. These techniques provide data on both aspects for a knee joint. However, since the work with natural and normal joints in this field is mainly related to locomotion studies [12], the results are affected by skin artefacts and measurement errors.

The most advanced results in anatomical-functional analysis of the knee joint at present are obtained by dynamic magnetic resonance imaging (MRI) [24] and Roentgen Stereophotogrammetric Analysis (RSA), which have been used in numerous experimental and in vivo studies [13, 14, 15]. However their accuracy in anatomical measurements of bone surfaces and soft tissues is not as good as with dissection methods and the motion tracking is complicate, time consuming and exposed to radiation (that is low only in MRI).

Other methods determine the anatomical coordinates of surface points of the organs of a joint by means of pointing systems:

- EP 0603089, is specific to finding points in femur and tibia that are invariant with respect to any movement of the two organs of a joint, but cannot be used for mapping all the positions of the two organs of a joint;
- EP 581704 describes how to obtain a cloud of points of an organ by a pointing system and then how to combine the image of the cloud of points with a second image of the organ obtained by another system, such as an echographic system.

However, the prior art does not provide a combination of the images of two organs and then to obtain a motion tracking with respect to each other.

Summary of the invention

It is, then, object of the present invention, to provide a method for mapping two organs of a joint, such as femur and tibia at the knee joint as well as the organs of hip, shoulder, elbow, ankle, wrist joints, etc., that is capable to reproduce the real joint in a controllable and measurable environment and can quantify both anatomical and functional features thereof, as well as the correlation to each other.

It is another object of the present invention to provide a method for mapping a joint that allows to track anatomical structures during motion with numerical and statistical evaluation of sections and views thereof comprising bone, ligaments insertions and interpolated points.

These objects are achieved by the method according to the invention, for simultaneous anatomical and functional mapping of at least a first and a second organ of a joint, characterised in that it comprises the steps of:

- providing an electrogoniometer having an end with a plurality of degrees of freedom, the electrogoniometer being associated to a CPU for storing and computing angular and spatial coordinates with respect to a fixed reference system;
- fastening to the end a probe, the electrogoniometer being triggered to determine the coordinates of points touched by the probe;
- acquiring by the electrogoniometer the anatomical coordinates of a plurality of surface points of the both first and second organ by locating the probe at the points and computing the surface of the first and second organ starting from the surface points;
- acquiring by the electrogoniometer the coordinates of at least three not aligned landmarks of both the first and second organ by locating the probe at the landmarks, the surface points being associated to a reference system integral to the landmarks ;
- fastening the end of the electrogoniometer to the first organ by means of a tracking support so that both the tracking support and the first organ move integrally to the end, a matching step being provided for associating the tracking support to the reference system integral to the landmarks ;
- moving the first organ with respect to the second organ and acquiring by the electrogoniometer a functional spatial sequence of positions of the tracking support, the spatial sequence being stored by the CPU;
- combining by the CPU the anatomical positions to

the spatial sequence of positions and mapping automatically a sequence of mutual anatomical-functional positions of the organs.

The spatial sequence acquisition may be carried out prior or later than the anatomical acquisition. If the functional spatial sequence acquisition is the former, the tracking support is mounted at the end of the electrogoniometer, the first organ is moved and the sequence is recorded. Then, the tracking support is removed and replaced with the probe, by which both organs are scanned separately or in a fixed mutual position and their anatomical coordinates recorded. The landmarks of each organ are then acquired for matching the anatomical coordinates with the spatial sequence.

If the anatomical acquisition is the former, by the probe both organs are scanned separately or in a fixed mutual position and their anatomical coordinates recorded. The landmarks of each organ are then acquired. The electrogoniometer is blocked in all movements and the probe is replaced by the tracking support, that is fastened to the first organ. Before freeing the electrogoniometer, the coordinates of at least one point of the tracking support are recorded as a matching step. Then, the first organ is moved and the spatial sequence is recorded.

According to another aspect of the invention, an apparatus for simultaneous anatomical and functional mapping of two organs of a joint comprises:

- an electrogoniometer having an end with a plurality of degrees of freedom,
- a CPU associated to the electrogoniometer for storing and computing angular and spatial coordinates of the end and of points to it associated with respect to a fixed reference system;
- a probe that can be fastened at the end;
- software means associated to the CPU for acquiring by the electrogoniometer the coordinates of a plurality of surface points of the both first and second organ scanned by the probe and for computing the surface of the first and second organ starting from the surface points,
- a plurality of not aligned landmarks associated to the first and second organ,
- a tracking support to be fixed integral to the first organ and to the end of the electrogoniometer so that both a part of the electrogoniometer and the first organ move integrally to each other,
- means for matching the tracking support and the landmarks, so that the surface of the first and second organs are associated to the electrogoniometer,
- software means residing in the CPU for recording the positions of the electrogoniometer and for determining and mapping the positions of all the points of the first organ with reference to the second organ.

Said electrogoniometer preferably provides six coordinates for each spatial position of its end.

A software means associated to the CPU carries out the following steps:

- Acquiring anatomical data of said organs by said probe as anatomical files of three spatial coordinates;
- Acquiring the coordinates of the landmarks of said organs as files of three coordinates;
- Acquiring functional positions as a sequence of movement files of six coordinates;
- Acquiring the coordinates of a point of said tracking support as a file of six coordinates,
- Computing a matching matrix as vectorial product of the files of the landmarks and the file of the tracking support, said matching matrix being a translation-rotation matrix of the reference system associated to said landmarks and the reference system associated to said tracking support;
- computing "structure" files as a product of the matching matrix and the spatial coordinates of the anatomical files;
- computing a display output as the product of the movement files and the structure files.

Preferably, the files of three spatial coordinates are obtained by a step of filtering three angular coordinates from files of six coordinates as resulting from the output of said electrogoniometer.

<u>Brief description of the drawings</u>

Further characteristics and the advantages of the method according to the invention will be made clearer with the following description of some of its embodiments, exemplifying but not limitative, with reference to the attached drawings, wherein:

- figure 1 shows a step of anatomical acquisitions of an organ by means of an apparatus according to the invention;
- figure 2 shows a step of kinematic data acquisition on a knee joint by means of the apparatus of figure 1;
- figure 3 shows a matching step on a knee joint by means of the apparatus of figure 1;
- figures 4A to 4E show a plot of the displayed acquisition of femur and tibia as well as of the ligaments as elaborated by the software in five positions of the a knee joint;
- figure 5 shows a 2D profile of a joint as displayed by the software starting from the computed coordinates.

<u>Description of a preferred embodiment</u>

With reference to figures 1 to 3, according to

the invention, for simultaneous anatomical and functional mapping of femur F and tibia T at a knee joint K, a probe 1 associated to an electrogoniometer 2 with an end 2a are provided.

The used equipment 2 is a commercial electrogoniometer, such as that produced by FARO Technologies. Electrogoniometer 2 has an anthropomorphic structure, three revolute links at the "wrist" 3 concurrent and mutually perpendicular, one at the "elbow" 4 and two at the "shoulder" 5.

Electrogoniometer 2 can acquire data continuously at 50 Hz rate (stream mode) or point by point under the user's trigger (point mode) an has a good accuracy, e.g. 0.3 mm / 0.3° accuracy in 1.8 m spherical workspace around its basement. It is light (several kgs) and flexible, and is mounted on an heavy and stable base 7 which is used to fasten it to an experimental desktop 8 and easily move it when necessary. Electrogoniometer 2 stores locations as files of six coordinates, i.e. x,y,z coordinates of probe 1 tip as well as Eulero angles in the sequence Z-X'-Z'' of its last link.

Electrogoniometer 2 has a control box connected with a standard PC and the relative CPU, not shown, via a serial port for storing and computing the coordinates of probe 1. A user-friendly, windows-like software may be used to let the user 6 acquire locations in ASCII files, and to set via software the sampling rate of motion, the acquisition mode (stream or point), to choose pre-calibrated end-effectors and any user-defined coordinate system.

The protocol for anatomical-functional acquisitions consists in two acquisition steps, and a matching phase.

The anatomical acquisitions are performed using electrogoniometer 2 equipped with sharp point probe 1 and digitising points on a target structure, such as a femur or a tibia, previously fixed to the desktop (Figure 1). The anatomical structure, for example a tibia T, is implanted with three reference and non collinear landmarks which can be small screws or pins $T_1$, $T_2$, $T_3$. They are acquired before the points on the surface each time that the anatomical structure T is moved to a different acquisition location.

The motion of the knee is acquired fixing electrogoniometer end 2a to a mobile bone segment F by a tracking support 10, i.e. a custom-made tool mounted on electrogoniometer "wrist" 3. For example, tool 10 is a modified short arm with an external fixator 11 (Figure 2). Once electrogoniometer 2 is rigidly fixed to the mobile bone segment F, its motion can be recorded each time the user triggers a sensor's acquisition button (not shown) or is sampled until the user releases it with a user's defined rate (< 50 Hz).

Each time the user switches from anatomical to functional acquisitions or vice versa, the bone F has to be kept still. When end 2a of electrogoniometer 2 is equipped with tracking support attached to bone F, before moving it for acquiring a spatial sequence of positions of the bone F, a matching step is performed by acquiring the coordinates of 1 point (whichever) of the tracking support same. On the other hand, when end 2a of electrogoniometer 2 is equipped with the point probe 1 (figure 3) the landmarks are digitised before or after digitising with the probe all the surface of an organ to it associated.

The recorded data, all in the form of files of six coordinates, are elaborated on-line or off-line by a dedicated software, of which a synthesis of the operation is the following. In the exemplifying embodiment anatomical and functional data are processed by the software that is written for example in MATLAB language, suitable for precise anatomical interpolations, for kinematic elaboration and a user-friendly interface for medical users.

For a knee joint, the input data of the program are the following.

- Bone surfaces and anatomical data: each rigid structure, such as tibia T or femur F, but also ligaments' attachment areas or epicondyles, are separate objects. The same anatomical structure is reconstructed as a unique cloud of points (eventually filtering any outliers) even if acquired in multiple positions, using an algorithm based on the single value decomposition [16, 17] to compute the transformation between the different reference landmarks (referred to as "SVDM").
- Trajectories: Files of locations of the mobile segment (and relative anatomical structures) are transformed into homogeneous coordinates roto-translation matrices.
- Display frame: an acquisition coordinate system used for displaying the joint is used which is usually chosen on the fixed bone at the extension position, following the clinical conventions on axes (Y axis in anterior-posterior direction, Z as the "vertical" tibial axis) and normalising non orthogonal relationships. The program allows the definition of an optional file containing the "adjustment" to the acquisition frame which meets the user's need. For example making posterior femoral condyles coincide in the lateral view, showing perfectly horizontal tibial plateau in the frontal view, or setting the origin on the tibial spine. The optional file is stored as a roto-translation around the acquisition axes.

The numerical elaboration provided by the program can be summarised into the following groups.

<u>Reconstruction of the joint during recorded motion</u>:

The program shows the 3D anatomical structures during recorded motions, allowing the examination of the successive positions of all or selected objects during the recorded trajectories (*Figures 4A-4E*). The positions of an anatomical structure during motion is computed from motion and anatomical input data according to the fol-

lowing formula:

$$\forall P \in S \qquad P_I = M_I \times M_0^{-1} \times F_0 \times (P) \qquad (1)$$

$$S_I = \{P_I\}_I$$

wherein

$S$ is the cloud of points describing the examined structure;

$P$ is a point of the cloud $S$ of points belonging to a surface of an organ

$M_0$ is the location of the recorded trajectory used during the matching phase (usually the first, e.g. full extension during the passive range of motion);

$M_i$ is the i<sup>th</sup> -location of the recorded motion;

$F_0$ is a SVDM - transformation from the acquisition position of the examined anatomical structure into the matching position;

$M_0^{-1} \times F_0 \times (P)$ is a structure file calculated for each point $P$

$P_i$ is the position calculated for each point $P$ at the i<sup>th</sup> instant of the examined motion

$S_i$ is the position of the mobile structure of points $P_i$ at the i<sup>th</sup> instant of the examined motion.

The software that carries out the above computations, substantially, operates according to the following steps:

- the anatomical data of the organs are acquired by said probe as anatomical files of six coordinates that are reduced into files P of three spatial coordinates;
- the landmarks of said organs are acquired as files of six coordinates $M_o$;
- functional positions of the mobile organ of the joint are acquired as a sequence of movement files $M_i$ of six coordinates;
- the reference system of the coordinates of a point of said tracking support is acquired as a file $F_o$ of six coordinates,
- a matching matrix $M_0^{-1} \times F_0$ is computed as vectorial product of the files of the landmarks and the file of the tracking support, said matching matrix being a translation-rotation matrix of the reference system associated to said landmarks and the reference system associated to said tracking support;
- "structure" files $M_0^{-1} \times F_0 \times (P)$ are computed as a product of the matching matrix and the spatial coordinates of the anatomical files;
- a display output of the cloud $S_i$ of points $P_i$ is obtained as the product $P_I = M_I \times M_0^{-1} \times F_0 \times (P)$ of the movement files $M_i$ and the structure files.

In Figures 4A-4E the position of the organs T and F are shown as calculated by the program as five clouds $S_i$.

A peculiar aspect of the program including both anatomy and motion is the possibility to track contact areas and points during selected trajectories, such as the ligaments insertions during PROM.

Anatomical computations - The examination of each structure can be performed in a 3D window in clinical views (frontal and lateral view) or from arbitrary angles and distances, like in a virtual spatial manipulation of the object. However more precise measurements are possible in sections of the joint at user's defined positions and orientation, computed according to the following formulas (2) and (3)

$$F = R_Z \times R_Y \times R_X \times F_S \qquad (2)$$

wherein

$F_S$ is the reference frame associated to the section plane chosen by the user.

In particular, being $N$ a vector normal to the section plane

if sagittal section

$$N = \begin{bmatrix} 1 \\ 0 \\ 0 \end{bmatrix}, \quad F_S = \begin{bmatrix} 0 & 0 & 1 \\ 1 & 0 & 0 \\ 0 & 1 & 0 \end{bmatrix};$$

if frontal section

$$N = \begin{bmatrix} 0 \\ 1 \\ 0 \end{bmatrix}, \quad F_S = \begin{bmatrix} 1 & 0 & 0 \\ 0 & 0 & -1 \\ 0 & 1 & 0 \end{bmatrix};$$

if coronal section

$$N = \begin{bmatrix} 0 \\ 0 \\ 1 \end{bmatrix}, \quad F_S = \begin{bmatrix} 1 & 0 & 0 \\ 0 & 1 & 0 \\ 0 & 0 & 1 \end{bmatrix};$$

$$R_X = \begin{bmatrix} 1 & 0 & 0 \\ 0 & \cos(\alpha) & sin(\alpha) \\ 0 & -sin(\alpha) & \cos(\alpha) \end{bmatrix};$$

$$R_Y = \begin{bmatrix} \cos(\beta) & 0 & sin(\beta) \\ 0 & 1 & 0 \\ -sin(\beta) & 0 & \cos(\beta) \end{bmatrix};$$

$$R_Z = \begin{bmatrix} \cos(\gamma) & sin(\gamma) & 0 \\ -sin(\gamma) & \cos(\gamma) & 0 \\ 0 & 0 & 1 \end{bmatrix};$$

is the user's defined rotation of the section plane around the X axis of the anatomical reference frame;

β is the user's defined rotation of the section plane around the Y axis of the anatomical reference frame;

γ is the user's defined rotation of the section plane around the Z axis of the anatomical reference frame;

= 0, β = 0, γ = 0 define standard sagittal, frontal and coronal section;

$$\forall P \in S \qquad P' = F^{-1} \times (P - P_S) \qquad (3)$$

if

$$|P'_Z| \le t/2$$

where $|P'_Z|$ is the absolute value of P' third co-ordinate, then $P' \in S_S$ $[P'_X \ P'_Y] \in S_{Profile}$

wherein

*F* is the reference frame associated to the user's defined section plane (defining its orientation);

$P_s$ one point of the user's defined section plane (defining its position on the joint);

*t* is the user's defined section thickness;

*S* is the cloud of points describing the examined structure;

$S_s$ is the 3D slice of S around the chosen section plane;

$S_{Profile}$ is the 2D curve describing the S profile in the chosen section plane;

Successive sagittal sections are possible by scanning the femoral condyles, frontal sections of the tibial plateaux or 3 mm coronal slices like in standard MRI examinations. The program provides also numerical algorithms for the least square fitting of the whole profiles or selected subsets with lines, circles or ellipses.

Kinematic computations - The availability of motion and anatomical data allows the computation of all the kinematic descriptions proposed in the literature, both based on anatomical decompositions [18, 19, 20, 21] or purely kinematic computations [22, 23]. In the present implementation we can compute instantaneous Euler angles in the chosen anatomical frame (sequence X -Y' - Z'') and instantaneous helical axes/angles.

According to the invention, the interaction of anatomical structures during motion can be studied in very natural conditions, as the passive motion can be acquired not only as a quasi-static collection of fixed positions (like in most MRI techniques) but also during the classical clinical movements, like in very recent fluoroscopic or optical studies with less accurate anatomical descriptions. In the described methodology both the acquisition procedure and the graphical presentation of measured data and elaboration are straightforward, easily repeatable, with a known numerical reliability and interactively adaptable to the specific study.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

REFERENCES

**[01]** Freeman to appear in JBJS (Nov 2000) - Anatomy Review

**[02]** S. Hirokawa, Biomechanics of the knee joint: a critical review, in Crit. Rev. Biomed. Eng., 21(2): 79-135, (1993).

**[03]** Pio, L. Carminati, G. Stennardo, L. Pedrotti, Evoluzione dei concetti di anatomia funzionale dell'articolazione femoro-tibiale (Evolution of the concepts of functional anatomy of the kneejoint), in Chir. Organi Mov., LXXXIII, pp. 401-411 (Cappelli Editore, Bologna, Italy, 1998).

**[04]** Y. Ando, H. Fukatsu, T. Ishigaki, I. Aoki, T. Yamada, Analysis of knee movement with low-field MR equipment-a normal volunteer study, in Radiat. Med., 12(4): 153-60, (1994).

**[05]** M.S. Hefzy, B.P. Kelly, T.D. Cooke, A.M. al-Baddah, L. Harrison, Knee kinematics in-vivo of kneeling in deep flexion examined by bi-planar radiographs, in Biomed. Sci Instrum., Vol. 33, pp. 453-8, (1997).

**[06]** R. Huiskes, J. Kremers, A. de Lange, H.J. Woltring, G. Selvik, Th.J.G. van Rens, Analytical stereophotogrammetric determination of three-dimensional knee-joint geometry, in J. of Biomechanics, 18(8): 559-70, (1985).

**[07]** T.P. Quinn, C.D. Mote, A six degree-of-freedom acoustic transducer for rotation and translation measurements across the knee, in Journal of Biomechanical Engineering, Vol. 112, pp. 371-378, (1990).

**[08]** R.J. Minns, W.K. Walsh, J.A. Clarket, Techniques for measuring the static and dynamic properties of the patella, in J. Biomed. Eng., Vol. 11, pp. 209-14, (1989).

**[09]** H. Fujie, K. Mabuchi, Savio L-Y Woo, G.A. Livesay, S. Arai, Y. Tsukamoto, The use of robotics technology to study human kinematics: a new methodology, in J. Biomed. Eng., Vol. 115, pp. 211-7, (1993).

**[010]**L.J. Ruijven, M. Beek, E. Donker, T.M.G.J. van Eijden, The accurancy of joint surface models constructed from data obtained with an electromagnetic tracking device, in J. of Biomechanics, Vol. 33, pp. 1023-8, (2000).

**[011]**T.P. Quinn, C.D. Mote, A six degree-of-freedom acoustic transducer for rotation and translation measurements across the knee, Journal of Biomechanical Engineering, Vol. 112, pp. 371-378, (1990).

**[012]**T.P. Andriacchi, E.J. Alexander, Studies of human locomotion: past, present and future, in J. Biomec., Vol. 33, pp. 1217-224, (2000).

**[013]**M. Niitsu, M. Akisada, I. Anno, S. Miyakawa, Moving knee joint: technique for kinematic MR Imaging, in Radiology, 174(2): 569-70, (1990).

**[014]**T.L. Haut, M.L. Hull, S.M. Howell, Use of Roentgenography and Magnetic Resonance Imaging to predict meniscal geometry determined with a three-dimensional coordinate digitizing system, in J. Orthop. Res., 18(2): 228-37, (2000).

**[015]**J. Pauly, Abstract: real-time interactive MRI for cardiac applications, in Computer Aided Surgery, Vol. 5, pp. 133, (2000).

**[016]**S.K. Arun, S.D. Clostein, Least-Square Fitting of Two 3-D Point Sets, in IEEE Trans on PAMI (Pattern Analysis and Machine Intelligence), PAMI-9 (5): 698-700, (1987).

**[017]**R.J. Hanson, M.J. Norris, Analysis of measurements based on the singular value decomposition, SIAM J SCI STAT COMPUT, 2(3): 363 - 373, (1981).

**[018]**M.A. Lafortune, The use of intra-cortical pins to measure the motion of the knee during walking, Ph.D. thesis, Pennsylvania State University (1984).

**[019]**G.R. Pennock, K.J. Clark, An anatomy-based coordinate system for the description of the kinematic displacements in the human knee, in Journal of Biomechanics, Vol. 23, pp. 1209-1218, (1990).

**[020]**E.Y.S. Chao, Justification of triaxial goniometer for the measurement of joint rotation, in Journal of Biomechanics, Vol. 13, pp. 989-1006, (1980).

**[021]**E.S. Grood, W.J. Suntay, A joint coordinate system for the clinical description of three-dimensional motions: application to the knee, in Journal of Biomechanical Engineering, Vol. 105, pp. 136-144, (1983).

**[022]**R.A. Hart, C.D. Mote, H.B. Skinner, A finite helical axes as a landmark for kinematic reference of the knee, in Journal of Biomechanical Engineering, Vol. 113, pp. 215-222, (1991).

**[023]**Blankevoort, L.R. Huiskes, A. De Lange, Helical axes of passive knee joint motions, in Journal of Biomechanics. Vol. 23, pp. 1219-1229, (1990).

**[024]**V. Pinskerova, H. Iwaki, M. Freeman, The shape and relative movements of the femur and tibia in the unloaded cadaveric knee: a study using MRI as an anatomical tool, in Surgery of the knee, 3rd edition, eds J.N. Insall and W.N. Scott (Saunders Inc., Philadelphia USA in press).

**[025]**Y.Y. Dhaher, L.D. Scott, W.Z. Rymer, The use of basis functions in modelling joint articular surfaces: application to the knee joint, in J. Biomech., Vol. 33, pp. 901-907, (2000).

**[026]**S.G. Elias, M.A.R. Freeman, E.I. Gokcay, A correlative study of geometry and anatomy of the distal femur, in Clinical Orthopaedics and Related Research, Number 260, pp. 99-103, (1990).

## Claims

1. A method for simultaneous anatomical and functional mapping of two organs of a joint, such as femur and tibia at the knee joint as well as the organs of hip, shoulder, elbow, ankle, wrist joints, **characterised in that** it comprises the steps of:

- providing an electrogoniometer having an end with a plurality of degrees of freedom, said electrogoniometer being associated to a CPU for storing and computing angular and spatial coordinates with respect to a fixed reference system;
- fastening to said end a probe, said electrogoniometer being triggered to determine the coordinate of points touched by said probe;
- acquiring by said electrogoniometer the anatomical coordinates of a plurality of surface points of said both first and second organ by locating said probe at said points and computing the surface of said first and second organ starting from said surface points;
- acquiring by said electrogoniometer the coordinates of at least three not aligned landmarks of said both first and second organ by locating said probe at said landmarks, said surface points being associated to a reference system integral to said landmarks ;
- fastening said end of said electrogoniometer to said first organ by means of a tracking support so that both said tracking support and said first organ move integrally to said end, a matching step being provided for associating said tracking support to said reference system integral to said landmarks ;
- moving said first organ with respect to said second organ and acquiring by said electrogoniometer a functional spatial sequence of positions of said tracking support, said spatial sequence being stored by said CPU;
- combining by said CPU the anatomical positions to the spatial sequence of positions and mapping automatically a sequence of mutual anatomical-functional positions of said organs.

**2.** A method according to claim 1, wherein said functional spatial sequence acquisition is made prior than said anatomical acquisition, the steps being provided of:

- mounting said tracking support at said end of said electrogoniometer;
- acquiring one point of said tracking support for the matching step,
- moving said first organ and recording a spatial sequence;
- removing the tracking support and replacing it with said probe,
- acquiring said landmarks for said matching step;
- scanning each organ and recording their anatomical coordinates.

**3.** A method according to claim 1, wherein said anatomical acquisition is made prior than said functional spatial sequence acquisition, the steps being provided of:

- mounting said probe at said end of said electrogoniometer;
- acquiring said landmarks for said matching step;
- scanning each organ and recording their anatomical coordinates;
- blocking said electrogoniometer in all movements and replacing said probe is by said tracking support, that is fastened to said first organ;
- acquiring one point of said tracking support for the matching step;
- moving said first organ and recording a spatial sequence.

**4.** A method according to claim 1, wherein the following steps are provided:

- Acquiring anatomical data of said organs by said probe as anatomical files of three spatial coordinates;
- Acquiring the coordinates of the landmarks of said organs as files of three coordinates;
- Acquiring functional positions as a sequence of movement files of six coordinates;
- Acquiring the coordinates of a point of said tracking support as a file of six coordinates,
- Computing a matching matrix as vectorial product of the files of the landmarks and the file of the tracking support, said matching matrix being a translation-rotation matrix of the reference system associated to said landmarks and the reference system associated to said tracking support;
- computing "structure" files as a product of the matching matrix and the spatial coordinates of the anatomical files;
- computing a display output as the product of the movement files and the structure files.

**5.** A method according to claim 4, wherein the files of three spatial coordinates are obtained by a step of filtering three angular coordinates from files of six coordinates as resulting from the output of said electrogoniometer.

**6.** An apparatus for simultaneous anatomical and functional mapping of two organs of a joint, such as femur and tibia at the knee joint as well as the organs of hip, shoulder, elbow, ankle, wrist joints, **characterised in that** it comprises:

- an electrogoniometer having an end with a plurality of degrees of freedom,
- a CPU associated to said electrogoniometer for storing and computing angular and spatial coordinates of said end and of points to it associated with respect to a fixed reference system;
- a probe that can be fastened at said end;
- software means associated to said CPU for acquiring by said electrogoniometer the coordinates of a plurality of surface points of said both first and second organ scanned by said probe and for computing the surface of said first and second organ starting from said surface points,
- a plurality of not aligned landmarks associated to said first and second organ,
- a tracking support to be fixed integral to said first organ and to said end of said electrogoniometer so that both a part of said electrogoniometer and said first organ move integrally to each other,
- means for matching said tracking support and said landmarks, so that the surface of said first and second organ are associated to said electrogoniometer,
- software means residing in said CPU for recording the positions of said electrogoniometer and for determining and mapping the positions of all the points of the first organ with reference to the second organ.

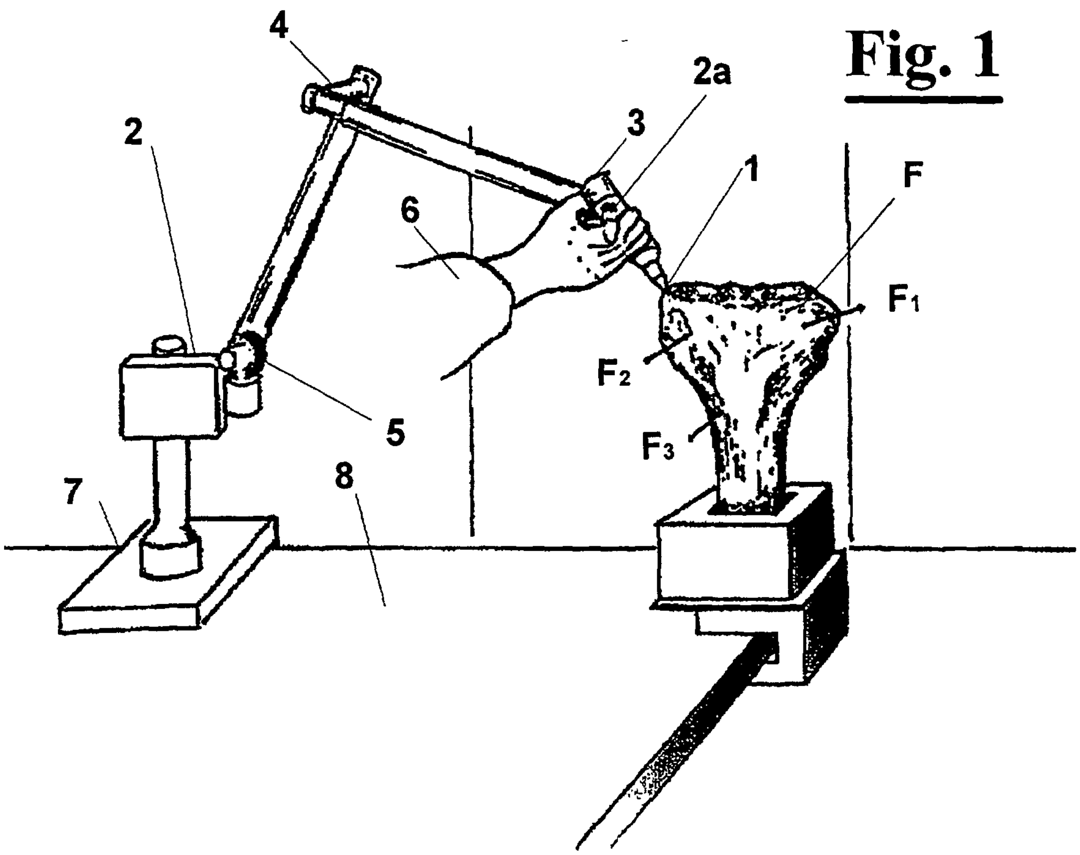


Fig. 1

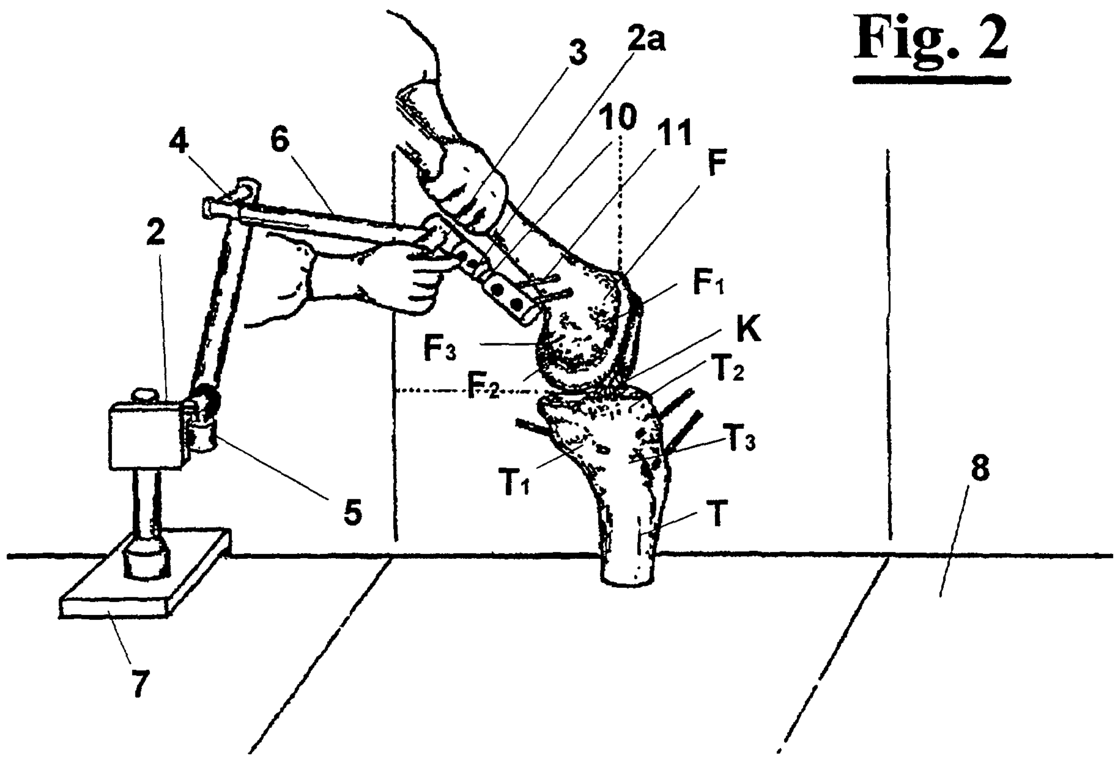


Fig. 2

Fig. 3

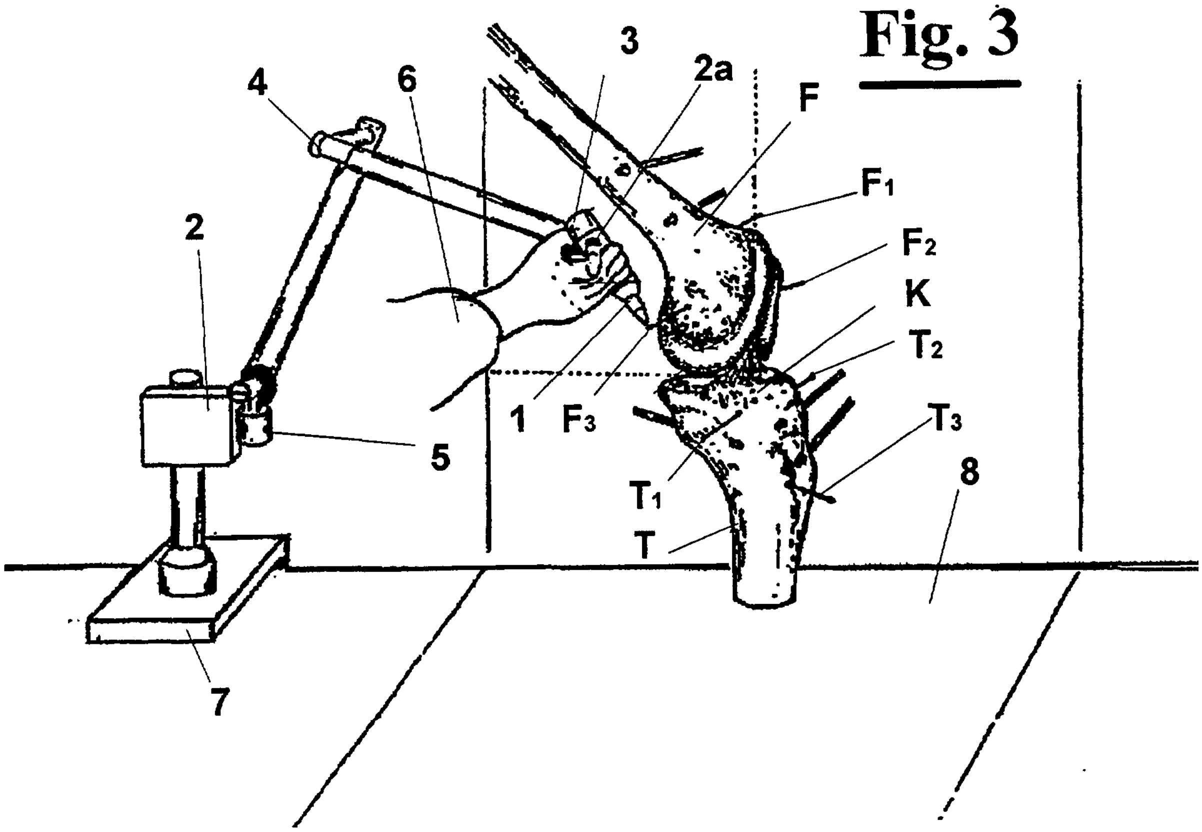


Fig. 4A

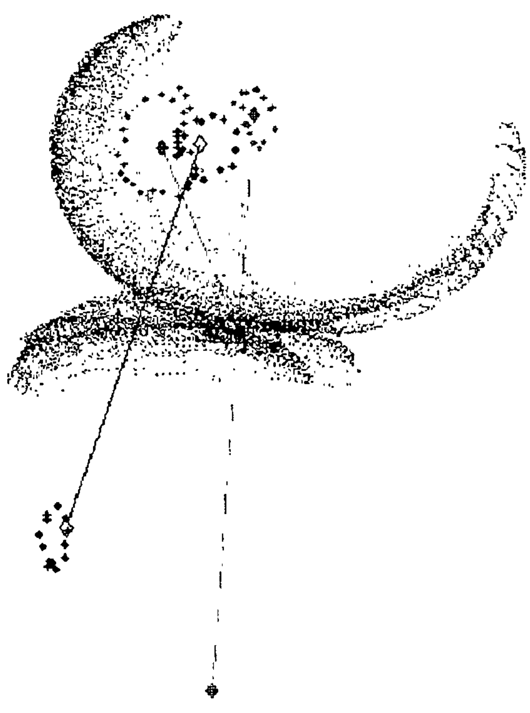

Fig. 4B

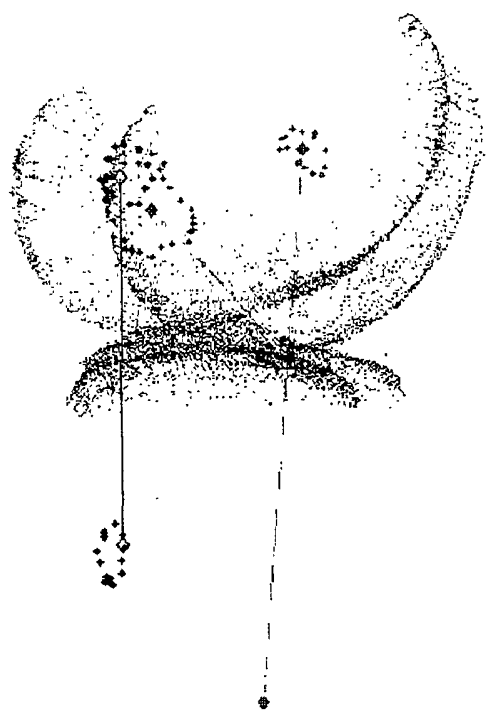

Fig. 4C

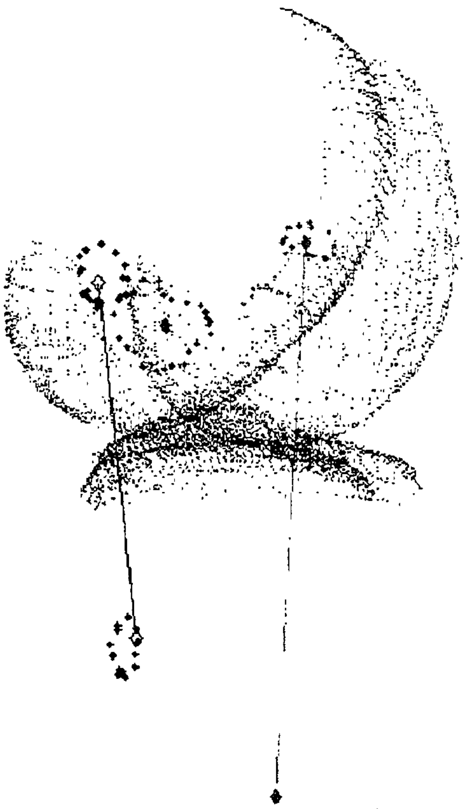

Fig. 4D

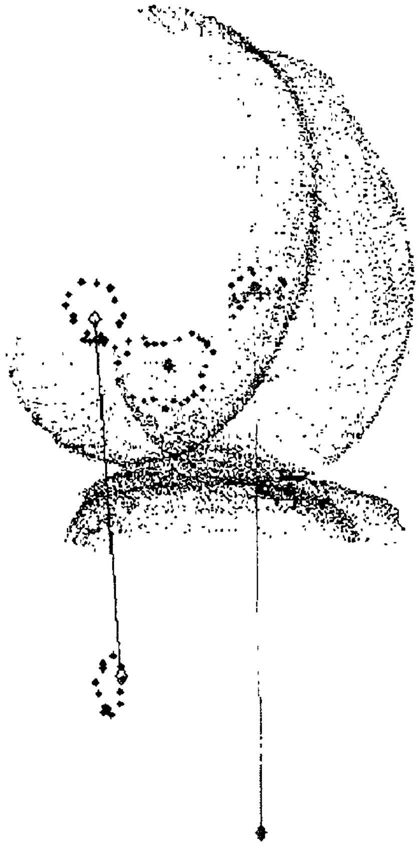

Fig. 4E

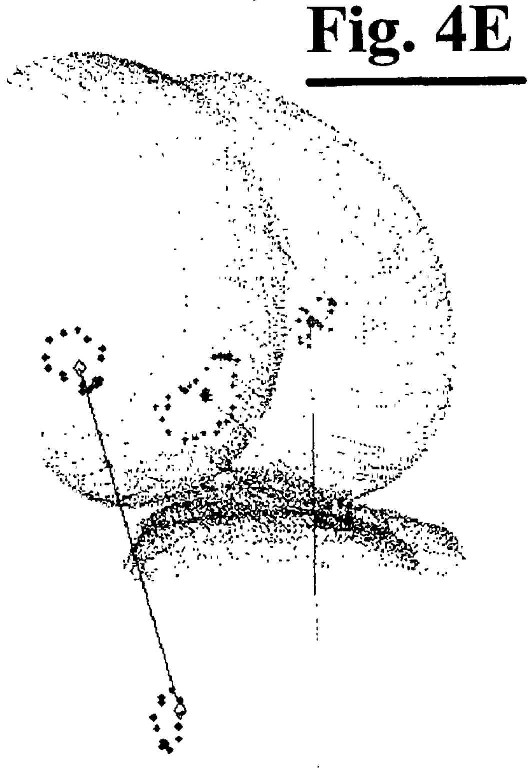

Fig. 5

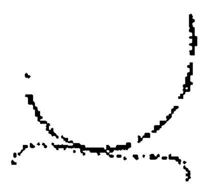

European Patent Office

# EUROPEAN SEARCH REPORT

Application Number
EP 00 12 8769

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 4 649 934 A (FRASER GREGORY A ET AL) 17 March 1987 (1987-03-17) * column 3, line 19 - column 4, line 50; figures * --- | 1-6 | A61B5/103 |
| A | US 5 251 127 A (RAAB SIMON) 5 October 1993 (1993-10-05) * column 3, line 10 - line 68 * --- | 1-6 | |
| A | AT 399 273 B (TRUPPE MICHAEL) 25 April 1995 (1995-04-25) * the whole document * ----- | 1 | |
| | | | TECHNICAL FIELDS SEARCHED (Int.Cl.7) |
| | | | A61B |

The present search report has been drawn up for all claims

| Place of search | Date of completion of the search | Examiner |
|---|---|---|
| THE HAGUE | 25 May 2001 | Manschot, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 12 8769

This annex lists the patent family membersrelating to the patent documents cited in the above-mentioned European search report.
The members are as contained in the European Patent Office EDP file on
The European Patent Office is in no way liable for these particulars which are merely given for the purpose of information.

25-05-2001

| Patent document cited in search report | Publication date | Patent family member(s) | Publication date |
|---|---|---|---|
| US 4649934 A | 17-03-1987 | AT 57826 T | 15-11-1990 |
| | | DE 3675266 D | 06-12-1990 |
| | | EP 0204639 A | 10-12-1986 |
| | | JP 62057543 A | 13-03-1987 |
| US 5251127 A | 05-10-1993 | CA 1336451 A | 25-07-1995 |
| | | AT 126994 T | 15-09-1995 |
| | | DE 69112538 D | 05-10-1995 |
| | | DE 69112538 T | 21-03-1996 |
| | | EP 0469966 A | 05-02-1992 |
| | | US 5748767 A | 05-05-1998 |
| | | EP 0326768 A | 09-08-1989 |
| | | JP 1280449 A | 10-11-1989 |
| | | JP 2930314 B | 03-08-1999 |
| | | US 5305203 A | 19-04-1994 |
| AT 399273 B | 25-04-1995 | AT 239890 A | 15-09-1994 |
| | | AT 133550 T | 15-02-1996 |
| | | CA 2056105 A | 27-05-1992 |
| | | DE 59107344 D | 14-03-1996 |
| | | EP 0488987 A | 03-06-1992 |
| | | JP 4336048 A | 24-11-1992 |
| | | US 5678546 A | 21-10-1997 |
| | | US 5823958 A | 20-10-1998 |

EPO FORM P0459

For more details about this annex : see Official Journal of the European Patent Office, No. 12/82